# EUROPÄISCHE PATENTSCHRIFT

(11) **EP 1 413 259 B1**
(45) Veröffentlichungstag und Bekanntmachung des Hinweises auf die Patenterteilung: **19.03.2008**
(21) Anmeldenummer: 03022430.7
(22) Anmeldetag: 07.10.2003
(51) Int. Cl.: A61C 13/00, A61C 13/087, A61K 6/08, A61K 6/083

(54) **Dentales Formteil, insbesondere künstlicher Zahn**
Dental moulded part, preferably artificial tooth
Pièce moulée dentaire, en particulier dent artificièlle

(30) Priorität: 23.10.2002 DE 10249518
(43) Veröffentlichungstag der Anmeldung: 28.04.2004
(73) Patentinhaber: Heraeus Kulzer GmbH, 63450 Hanau (DE)
(72) Erfinder: Savic, Novica, 63691 Ranstadt (DE); Stange, Frank, 88085 Langenargen (DE)
(74) Vertreter: Kühn, Hans-Christian

(56) Entgegenhaltungen:
- EP-A- 1 226 807
- DE-A- 3 906 673
- GB-A- 1 143 502
- US-A- 2 895 050
- US-A- 4 433 959
- US-A- 4 645 455

## Beschreibung

Die Erfindung betrifft ein dentales Formteil, insbesondere einen künstlichen Zahn, ein Verfahren zur Herstellung dieses Formteiles sowie eine Verwendung.

Natürliche Zähne weisen einen relativ hohen Grad an Fluoreszenz auf. Diese Leuchtkraft ist besonders stark bei sogenanntem Schwarzlicht sichtbar. Werden künstliche Zähne neben einen natürlichen Zahn gestellt, fallen erstere bei bestimmten Lichtverhältnissen durch eine erheblich geringere Leuchtkraft auf und wirken unnatürlich.

Im allgemeinen ist es bei künstlichen Zähnen üblich, Fluoreszenzmittel in die jeweiligen Materialmischungen der äußeren Schneideschicht zu geben, um anschließend den Zahn zu formen. Die Fluoreszenzmittel dienen dazu, das natürliche Aussehen des Zahns zu erhöhen.

Laut US 2,895,050 kann der Zusatz von Uranoxid, Ceroxid und ggf Vanadiumoxid und Samariumoxid die Fluoreszenz von Keramikzähnen verbessern. Die Menge der Fluoreszenzmittel kann in den einzelnen Schichten der Keramikzähne variieren.

US 4,170,823 offenbart einen künstlichen Keramikzahn, der eine Kombination von Terbium- und Cersalzen enthält, um eine gewünschte Fluoreszenz zu erzeugen.

Nachteilig hieran ist jedoch die Tatsache, dass in vielen Fällen ein Aussehen erhalten wird, das den heutigen hohen ästhetischen Anforderungen nicht gerecht wird.

Problem der vorliegenden Erfindung ist es daher, ein dentales Formteil, insbesondere einen künstlichen Zahn bereitzustellen, der hinsichtlich seiner Fluoreszenz ein wesentlich natürlicheres Aussehen vermittelt.

Dieses Problem wird durch ein dentales Formteil, insbesondere einen künstlichen Zahn, nach Anspruch 1, ein Verfahren zur Herstellung eines solchen Formteils, insbesondere eines künstlichen Zahnes, nach Anspruch 9 sowie durch eine Verwendung nach Anspruch 10 gelöst.

Beim erfindungsgemäßen dentalen Formteil, insbesondere künstlichem Zahn, mit einer äußeren Schneideschicht und einer darunterliegenden Dentinschicht, ist zwischen Dentin- und Schneideschicht eine fluoreszierende Materialschicht angeordnet. Dieser Aufbau trägt völlig überraschend entscheidend dazu bei, dass das Aussehen solcher insbesondere künstlicher Zähne eine ausgesprochen hohe Natürlichkeit vermittelt.
Darüber hinaus wird bei den erfindungsgemäßen künstlichen Zähnen sogar die Farbwirkung positiv beeinflusst.
Darüber hinaus ist es auch möglich, die fluoreszierende Materialschicht nicht gleichmäßig stark auszubilden, um eine gewisse Unregelmäßigkeit und damit eine Erhöhung der Natürlichkeit durch eine nicht völlig gleichmäßige Fluoreszenz zu erreichen (der natürliche Zahn fluoresziert auch nicht gleichmäßig).
Schließlich werden die in der fluoreszierenden Materialschicht enthaltenden Fluoreszenzmittel durch die über dieser angeordneten Schneidschicht (Schmelzschicht) mechanisch und chemisch geschützt, so dass eine wesentlich erhöhte Wirksamkeitsdauer auf der einen und eine bessere Verträglichkeit für den Patienten auf der anderen Seite zu konstatieren ist.

Beim dentalen Formteil setzt sich die fluoreszierende Materialschicht zusammen aus 5,00 - 95 Gewichts-% Monomer, 0,5 - 90 Gewichts-% Vernetzer, 0,1 - 1 Gewichts-% Initiator und 0,01 - 30 Gewichts-% Additiv, insbesondere enthält die fluoreszierende Materialschicht bis zu 40 Gewichts-% Perlpolymer und/oder bis zu 2 Gewichts-% Pigment.
In der Praxis haben sich die folgenden Ausgestaltungen insbesondere hinsichtlich Fluoreszenzwirkung und Verarbeitbarkeit als vorteilhaft erwiesen.
Beim Monomer handelt es sich um mindestens eines aus der folgenden Gruppe: ethylenisch ungesättigte Monomere, monofunktionelle oder polyfunktionelle Acrylate und -Methacrylate, Alkylmethacrylate, Methylmethacrylat, Ethylmethacrylat, iso- und n-Butylmethacrylat, n-Hexylmethacrylat, Ethylhexylmethacrylat, Hydroxyethylmethacrylat.

Beim Vernetzer handelt es sich um mindestens einen aus der folgenden Gruppe: mehrfunktionelle Methacrylate und mehrfunktionelle Acrylate, Tetraethylenglykoldimethacrylat, Triethylenglykoldimethacrylat, Diethylenglykoldimethacrylat, Ethylenglykoldimethacrylat, Polyethylenglykoldimethacrylat, Butandioldimethacrylat, Hexandioldimethacrylat, Decan- und Dodecandioldimethacrylat, Bis-GMA, Bis-GA, Trimethylolpropantrimethacrylat, Produkte der Reaktion von Isocyanaten, Di- oder Triisocyanaten, mit Hydroxymethacrylaten oder -Acrylaten, Pentaerythritoltetraacrylate.

Beim Perlpolymer handelt es sich um mindestens eines aus der folgenden Gruppe:
bestehend aus Polymethylmethacrylat oder Copolymer, mit einer Korngröße von 5 - 70 µm und einer Molmasse zwischen 400.000 und 900.000 (g/mol), wobei es sich beim Copolymer um Allylmethacrylat, Ethylmethacrylat, Ethylhexylmethacrylat, Methylacrylat, Methacrylsäure, iso- und n- Butylmethacrylat, Hexylmethacrylat, Butandioldimethacrylat, Ethylenglykoldimethacrylat handelt.

Beim Initiator handelt es sich um mindestens einen aus der folgenden Gruppe: Peroxide, Dibenzoylperoxid, tertiäre Amine, Dimethylparatoluidin, wobei die Amine als Co-Initiatoren mit einer Menge von bis zu 0,5 Gewichts-% fungieren können.

Beim Pigment handelt es sich um mindestens eines aus der folgenden Gruppe: Titandioxid, Chromoxide, Antimonoxide, Eisenoxide, Kohlenstoff, Bariumsulfat, Azo-Ca-Salze, Nioxide, Azoverbindungen, Ultramarin, sowie gemischte Oxide der genannten Metalle.

Beim Additiv handelt es sich um mindestens eines aus der folgenden Gruppe: Fluoreszenzpigmente und/oder -Farbstoffe: benzoide und chinoide Aromaten und Heteroaromaten, Triarylmethane, Anthrachinone, Chromene, Xanthene, Indole, Chinoline, Acridine, Phenoxazine, Phenothiazine, Azo- und Stilbenfarbstoffe, Indigoderivate, Phthalocyanine, Tetrapyrrolfarbstoffe; optische Aufheller: Thiophenediyl-Benzooxazole, Stilbene-Benzooxazole, 7-Amino-4-methyl-coumarin, Dibenzopyridin, Azaanthrazene, Phenylendiamin, Naphtylamin, Coumarin, 7-Hydroxycoumarin;
Füllstoffe: pyrogenes Siliziumdioxid, hochfeines Titandioxid, mit Korngrößen kleiner 100 nm.

Schließlich ist es vorteilhaft, wenn die Korngröße der Füllstoffe etwa 10 nm beträgt, da eine besonders intensive Opaleszenz erreicht wird, die in Einzelfällen ästhetisch besonders hochwertige Ergebnisse ergibt.

Beim erfindungsgemäßen Verfahren zur Herstellung eines dentalen Formteils, insbesondere eines künstlichen Zahnes, insbesondere eines erfindungsgemäßen, wird zwischen Dentin- und Schneideschicht eine fluoreszierende Materialschicht angeordnet, beispielsweise mittels Aufsprühen oder Aufpinseln einer Monomer-Flüssigkeit mit Fluoreszenz-Partikeln auf die innere Seite der Schneideschicht vor dem Einlegen der Dentin- und schließlich inneren Halsschicht (der künstliche Zahn wird quasi in einer entsprechenden Negativform schichtweise von außen nach innen aufgebaut).

Nachfolgend wird die Erfindung anhand einer Zeichnung und eines Ausführungsbeispiels näher erläutert.

In der Zeichnung zeigt:

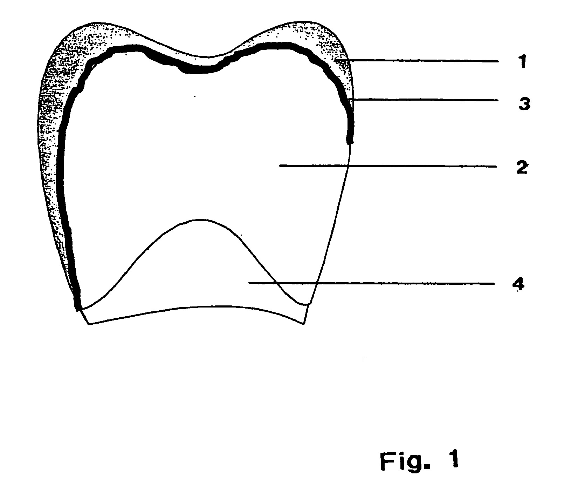

Figur 1 - eine skizzenhafte Querschnittsdarstellung eines Zahnes.

In Figur 1 ist skizzenhaft im Querschnitt ein Zahn dargestellt, der den erfindungsgemäßen Schichtaufbau aufweist. Auf einer inneren Halsschicht 4 befindet sich eine Dentinschicht 2, die wiederum im wesentlichen vollständig von einer äußeren Schneideschicht (Schmelzschicht) 1 umgeben ist. Zwischen der Schneideschicht (Schmelzschicht) 1 und der Dentinschicht 2 befindet sich eine fluoreszierende Zwischenschicht 3.

Die Herstellung eines künstlichen Zahnes mit einer entsprechenden Schichtfolge erfolgt in mehrteiligen Metallformen. Der schichtweise Aufbau beginnt mit der Schneideschicht (Schmelzschicht), wobei jede Schicht auf die vorherige aufgebracht und separat polymerisiert wird.

### Schneideschicht (Schmelzschicht):

Es wird ein übliches Zahnmaterial aus vernetztem PMMA (Polymethylmethacrylat) verwendet, wobei die Mischung wenig Pigmente enthält, um eine gute Farbwirkung zuzulassen. Primär als Pigment wird Titandioxid eingesetzt.

### Fluoreszierende/opaleszierende Zwischenschicht:

### Rezepturbeispiel 1:

| | | |
|---|---|---|
| Monomer | Methylmethacrylat | 36,16 % |
| Vernetzer | Ethylenglykoldimethacrylat | 50 % |
| Initiator | Benzoylperoxid | 0,4 % |
| | Dimethylparatoluidin | 0,4 % |
| Perlpolymer | Copolymer, enthaltend Ethylhexylacrylat (z.B. Polymer B611 der Firma Ineos) | 13 % |
| Additiv | Fluoreszenzmittel Lumilux Blau LZ | 0,04 % |

ergibt eine fluoreszierende Zwischenschicht.

### Rezepturbeispiel 2:

| | | |
|---|---|---|
| Monomer | Methylmethacrylat | 36,13 % |
| Vernetzer | Ethylenglykoldimethacrylat | 50 % |
| Initiator | Benzoylperoxid | 0,4 % |
| | Dimethylparatoluidin | 0,4 % |
| Perlpolymer | Copolymer, enthaltend Ethylhexylacrylat (z.B. Polymer B611 der Firma Ineos) | 13 % |
| Additiv | nanoteiliges Titandioxid, Teilchengröße 10 nm | 0,07 % |

ergibt eine opaleszierende Zwischenschicht.

### Herstellung und Anwendung der Lacke:

Monomere/Vernetzer werden vorgelegt und unter Rühren im geschlossenen Gefäß das Perlpolymer gelöst. Anschließend wird das Additiv zugegeben und ebenfalls durch Rühren dispergiert. Bei grobteiligen Additiven ist es sinnvoll diese vor Zugabe in etwas Vernetzer separat, beispielsweise durch Vermahlen, aufzuschließen.

Abschließend werden die Initiatorkomponenten direkt vor der Verwendung zugegeben. Das Auftragen auf die vorpolymerisierte Schneideschicht (Schmelzschicht) erfolgt vorteilhafterweise durch Applikation mit einem Pinsel. Durch Ablüften bzw. beginnende Polymerisation kommt es zu einer Verfestigung der Schicht, so dass ein Auftrag der nachfolgenden Dentinschicht problemlos möglich ist.

### Dentin- und Halsschicht:

Hierbei wird ein übliches Zahnmaterial aus vernetztem PMMA (Polymethylmethacrylat) verwendet, wobei die Mischung deutlich mehr Pigmente enthält, um eine gute Farbwirkung zu erzielen. Speziell das Dentin dient als Farbträger innerhalb des Zahnes.

## Patentansprüche

1. Dentales Formteil, insbesondere künstlicher Zahn, mit einer äußeren Schneideschicht (1) und einer darunterliegenden Dentinschicht (2), wobei zwischen Dentin- und Schneideschicht (2, 1) eine fluoreszierende Materialschicht (3) angeordnet ist,
**dadurch gekennzeichnet, dass** die fluoreszierende Materialschicht (3) sich zusammensetzt aus 5,00 - 95 Gewichts-% Monomer, 0,5 - 90 Gewichts-% Vernetzer, 0,1 - 1 Gewichts-% Initiator und 0,01- 30 Gewichts-% Additiv,
die fluoreszierende Materialschicht (3) bis zu 40 Gewichts-% Perlpolymer enthält, und/oder die fluoreszierende Materialschicht (3) bis zu 2 Gewichts-% Pigment enthält.

2. Dentales Formteil nach Anspruch 1, **dadurch gekennzeichnet, dass** es sich beim Monomer um mindestens eines aus der folgenden Gruppe handelt: ethylenisch ungesättigte Monomere, monofunktionelle oder polyfunktionelle Acrylate und -Methacrylate, Alkylmethacrylate, Methylmethacrylat, Ethylmethacrylat, iso- und n-Butylmethacrylat, n-Hexylmethacrylat, Ethylhexylmethacrylat, Hydroxyethylmethacrylat.

3. Dentales Formteil nach einem der Ansprüche 1 und 2, **dadurch gekennzeichnet, dass** es sich beim Vernetzer um mindestens einen aus der folgenden Gruppe handelt: mehrfunktionelle Methacrylate und mehrfunktionelle Acrylate, Tetraethylenglykoldimethacrylat, Triethylenglykoldimethacrylat, Diethylenglykoldimethacrylat, Ethylenglykoldimethacrylat, Polyethylenglykoldimethacrylat, Butandioldimethacrylat, Hexandioldimethacrylat, Decan- und Dodecandioldimethacrylat, Bis-GMA, Bis-GA, Trimethylolpropantrimethacrylat, Produkte der Reaktion von Isocyanaten, Di- oder Triisocyanaten, mit Hydroxymethacrylaten oder -Acrylaten, Pentaerythritoltetraacrylate.

4. Dentales Formteil nach Anspruch 1, **dadurch gekennzeichnet, dass** es sich beim Perlpolymer um mindestens eines aus der folgenden Gruppe handelt: bestehend aus Polymethylmethacrylat oder Copolymer, mit einer Korngröße von 5 - 70 µm und einer Molmasse zwischen 400.000 und 900.000, wobei es sich beim Copolymer um Allylmethacrylat, Ethylmethacrylat, Ethylhexylmethacrylat, Methylacrylat, Methacrylsäure, iso- und n-Butylmethacrylat, Hexylmethacrylat, Butandioldimethacrylat, Ethylenglykoldimethacrylat handelt.

5. Dentales Formteil nach einem der Ansprüche 1 bis 4, **dadurch gekennzeichnet, dass** es sich beim Initiator um mindestens einen aus der folgenden Gruppe handelt: Peroxide, Dibenzoylperoxid, tertiäre Amine, Dimethylparatoluidin.

6. Dentales Formteil nach Anspruch 1, **dadurch gekennzeichnet, dass** es sich beim Pigment um mindestens eines aus der folgenden Gruppe handelt: Titandioxid, Chromoxide, Antimonoxide, Eisenoxide, Kohlenstoff, Bariumsulfat, Azo-Ca-Salze, Nioxide, Azoverbindungen, Ultramarin, sowie gemischte Oxide der genannten Metalle.

7. Dentales Formteil nach einem der Ansprüche 1 bis 6, **dadurch gekennzeichnet, dass** es sich beim Additiv um mindestens eines aus der folgenden Gruppe handelt: Fluoreszenzpigmente und/oder -Farbstoffe: benzoide und chinoide Aromaten und Heteroaromaten, Triarylmethane, Anthrachinone, Chromene, Xanthene, Indole, Chinoline, Acridine, Phenoxazine, Phenothiazine, Azo- und Stilbenfarbstoffe, Indigoderivate, Phthalocyanine, Tetrapyrrolfarbstoffe; optische Aufheller: Thiophenediyl-Benzooxazole, Stilbene-Benzooxazole, 7-Amino-4-methylcoumarin, Dibenzopyridin, Azaanthrazene, Phenylendiamin, Naphtylamin, Coumarin, 7-Hydroxycoumarin;
Füllstoffe: pyrogenes Siliziumdioxid, hochfeines Titandioxid, mit Korngrößen kleiner 100 nm.

8. Dentales Formteil nach Anspruch 7, **dadurch gekennzeichnet, dass** die Korngröße der Füllstoffe etwa 10 nm beträgt.

9. Verfahren zur Herstellung eines dentalen Formteils nach einem der Ansprüche 1 bis 8, **dadurch gekennzeichnet, dass** zwischen Dentin- und Schneideschicht (2, 1) eine fluoreszierende Materialschicht (3) angeordnet wird.

10. Verwendung einer in den Ansprüchen 1 bis 8 aufgezeigten fluoreszierenden Materialschicht (3) als Schicht zwischen Dentin- und Schneideschicht (2, 1) eines dentalen Formteils, insbesondere eines künstlichen Zahnes.

## Claims

1. Dental moulding, in particular an artificial tooth, having an outer incisal layer (1) and an underlying dentin layer (2), a fluorescent material layer (3) being arranged between dentin layer and incisal layer (2, 1), **characterized in that** the fluorescent material layer (3) is composed to 5.00 - 95 % by weight of monomer, 0.5 - 90 % by weight of crosslinking agent, 0.1 - 1 % by weight of initiator and 0.01 - 30 % by weight of additive, the fluorescent material layer (3) contains up to 40 % by weight of bead polymer and/or the fluorescent material layer (3) contains up to 2 % by weight of pigment.

2. Dental moulding according to Claim 1, **characterized in that** the monomer is at least one from the following group: ethylenically unsaturated monomers, monofunctional or polyfunctional acrylates and methacrylates, alkyl methacrylates, methyl methacrylate, ethyl methacrylate, isobutyl and n-butyl methacrylate, n-hexyl methacrylate, ethylhexyl methacrylate and hydroxyethyl methacrylate.

3. Dental moulding according to either of Claims 1 or 2, **characterized in that** the crosslinking agent is at least one from the following group: polyfunctional methacrylates and polyfunctional acrylates, tetraethylene glycol dimethacrylate, triethylene glycol dimethacrylate, diethylene glycol dimethacrylate, ethylene glycol dimethacrylate, polyethylene glycol dimethacrylate, butanediol dimethacrylate, hexanediol dimethacrylate, decanediol dimethacrylate and dodecanediol dimethacrylate, bis-GMA, bis-GA, trimethylolpropane trimethacrylate, products of the reaction of isocyanates, diisocyanates or triisocyanates with hydroxymethacrylates or hydroxyacrylates or pentaerythrityl tetraacrylates.

4. Dental moulding according to Claim 1, **characterized in that** the bead polymer is at least one from the following group: consisting of polymethyl methacrylate or copolymer having a particle size of 5 - 70 µm and a molar mass between 400,000 and 900,000, the copolymer being allyl methacrylate, ethyl methacrylate, ethylhexyl methacrylate, methyl acrylate, methacrylic acid, isobutyl and n-butyl methacrylate, hexyl methacrylate, butanediol dimethacrylate or ethylene glycol dimethacrylate.

5. Dental moulding according to any of Claims 1 to 4, **characterized in that** the initiator is at least one from the following group: peroxides, dibenzoyl peroxide, tertiary amines, dimethylparatoluidine.

6. Dental moulding according to Claim 1, **characterized in that** the pigment is at least one from the following group: titanium dioxide, chromium oxides, antimony oxides, iron oxides, carbon, barium sulphate, azo calcium salts, nickel oxides, azo compounds, ultramarine and mixed oxides of said metals.

7. Dental moulding according to any of Claims 1 to 6, **characterized in that** the additive is at least one from the following group; fluorescent pigments and/or fluorescent dyes; benzoid and quinoid aromatics and heteroaromatics, triarylmethanes, anthraquinones, chromenes, xanthenes, indoles, quinolines, acridines, phenoxazines, phenothiazines, azo and stilbene dyes, indigo derivatives, phthalocyanines, tetrapyrrole dyes; optical brighteners: thiophenediylbenzoxazoles, stilbenebenzoxazoles, 7-amino-4-methylcoumarin, dibenzopyridine, azaanthracenes, phenylenediamine, naphthylamine, coumarin, 7-hydroxycoumarin; fillers: pyrogenic silica, highly disperse titanium dioxide, having particle sizes of less than 100 nm.

8. Dental moulding according to Claim 7, **characterized in that** the particle size of the fillers is about 10 nm.

9. Method for producing a dental moulding according to any of Claims 1 to 8, **characterized in that** a fluorescent material layer (3) is arranged between the dentin layer and the incisal layer (2, 1).

10. Use of a fluorescent material layer (3) identified in Claims 1 to 8 as a layer between the dentin layer and the incisal layer (2, 1) of a dental moulding, in particular of an artificial tooth.

## Revendications

1. Pièce moulée dentaire, en particulier dent artificielle, avec une couche tranchante externe (1) et une couche de dentine (2) située sous la précédente, où une couche de matière fluorescente (3) est disposée entre les couches de dentine et tranchante (2, 1), **caractérisée en ce que** la couche de matière fluorescente (3) se compose de 5,00 - 95 % en masse de monomère, 0,5 - 90 % en masse de réticulant, 0,1 - 1 % en masse d'initiateur et 0,01 - 30 % en masse d'additif, la couche de matière fluorescente (3) contient jusqu'à 40 % en masse de polymère en perles, et/ou la couche de matière fluorescente (3) contient jusqu'à 2 % en masse de pigment.

2. Pièce moulée dentaire selon la revendication 1, **caractérisée en ce que** le monomère est au moins l'un du groupe suivant : monomères éthyléniquement insaturés, acrylates et méthacrylates monofonctionnels ou polyfonctionnels, méthacrylates d'alkyle, méthacrylate de méthyle, méthacrylate d'éthyle, méthacrylate d'iso- et n-butyle, méthacrylate de n-hexyle, méthacrylate d'éthylhexyle, méthacrylate d'hydroxyéthyle.

3. Pièce moulée dentaire selon l'une des revendications 1 et 2, **caractérisée en ce que** le réticulant est au moins l'un du groupe suivant : méthacrylates polyfonctionnels et acrylates polyfonctionnels, diméthacrylate de tétraéthylèneglycol, diméthacrylate de triéthylèneglycol, diméthacrylate de diéthylèneglycol, diméthacrylate d'éthylèneglycol, diméthacrylate de polyéthylèneglycol, diméthacrylate de butanediol, diméthacrylate d'hexanediol, diméthacrylate de décane- et dodécanedol, bis-GMA, bis-GA, triméthacrylate de triméthylolpropane, produits de la réaction d'isocyanates, de di- ou triisocyanates, avec des hydroxyméthacrylates ou hydroxyacrylates, tétraacrylates de pentaérythritol.

4. Pièce moulée dentaire selon la revendication 1, **caractérisée en ce que** le polymère en perles est au moins l'un du groupe suivant : consistant en poly(méthacrylate de méthyle) ou copolymère, ayant une taille de grains de 5 - 70 µm et une masse molaire entre 400 000 et 900 000, le copolymère étant le méthacrylate d'allyle, le méthacrylate d'éthyle, le méthacrylate d'éthylhexyle, l'acrylate de méthyle, l'acide méthacrylique, le méthacrylate d'iso- et n-butyle, le méthacrylate d'hexyle, le diméthacrylate de butanediol, le diméthacrylate d'éthylèneglycol.

5. Pièce moulée dentaire selon l'une des revendications 1 à 4, **caractérisée en ce que** l'initiateur est au moins l'un du groupe suivant : peroxydes, peroxyde de dibenzoyle, amines tertiaires, diméthyl-paratoluidine.

6. Pièce moulée dentaire selon la revendication 1, **caractérisée en ce que** le pigment est au moins l'un du groupe suivant : dioxyde de titane, oxydes de chrome, oxydes d'antimoine, oxydes de fer, carbone, sulfate de baryum, sels de Caazoïques, oxydes de nickel, composés azoïques, outremer ainsi que les oxydes mixtes des métaux cités.

7. Pièce moulée dentaire selon l'une des revendications 1 à 6, **caractérisée en ce que** l'additif est au moins l'un du groupe suivant : pigments fluorescents et/ou colorants fluorescents : aromatiques et hétéroaromatiques benzoïdes et quinoïdes, triarylméthanes, anthraquinones, chromènes, xanthènes, indoles, quinoléines, acridines, phénoxazines, phénothiazines, colorants azoïques et de stilbène, dérivés de l'indigo, phtalocyanines, colorants de tétrapyrrole; azurants optiques : thiophènediyl-benzooxazoles, stilbène-benzooxazoles, 7-amino-4-méthylcoumarine, dibenzopyridine, azaanthracènes, phénylènediamine, naphtylamine, coumarine, 7-hydroxycoumarine; charges : dioxyde de silicium pyrogène, dioxyde de titane ultrafin, avec des tailles de grains inférieures à 100 nm.

8. Pièce moulée dentaire selon la revendication 7, **caractérisée en ce que** la taille des grains des charges est d'environ 10 nm.

9. Procédé de production d'une pièce moulée dentaire selon l'une des revendications 1 à 8, **caractérisé en ce qu'**une couche de matière fluorescente (3) est disposée entre les couches de dentine et la couche tranchante (2, 1).

10. Utilisation d'une couche de matière fluorescente (3) selon les revendications 1 à 8 comme couche entre les couches de dentine et tranchante (2, 1) d'une pièce moulée dentaire, en particulier d'une dent artificielle.
